# EUROPEAN PATENT APPLICATION

(11) **EP 3 888 713 A1**
(43) Date of publication of application: **06.10.2021**
(21) Application number: 20184783.7
(22) Date of filing: 08.07.2020
(51) Int. Cl.: A61L 27/36, A61L 27/44, A61L 27/50, A61L 27/52, A61L 27/54, A61L 27/56

(54) **BONE GRAFT COMPOSITION AND PREPARATION METHOD THEREFOR**

(30) Priority: 31.03.2020 KR 20200039213
(71) Applicant: MedPark Co., Ltd., Busan 46504 (KR)
(72) Inventor: BAK, JeongBok, 46509 Busan (KR)
(74) Representative: BCKIP

(57) **Abstract**

The present disclosure relates to a bone graft composition containing hydroxypropyl methylcellulose and a preparation method therefor. More specifically, a bone graft composition containing hydroxypropyl methylcellulose in an amount that provides optimum osmotic properties and shape retainability, and a preparation method therefor.

## Description

### CROSS-REFERENCE TO RELATED APPLICATION(S)

This application claims priority to Korean Patent Application No. 10-2020-0039213, filed on March 31, 2020, the disclosure of which is incorporated herein by reference in its entirety.

### BACKGROUND OF THE DISCLOSURE

### Technical Field

The present disclosure relates to a bone graft composition and a preparation method therefor.

### Description of the Related Art

Various materials and various methods may be used for reconstruction of defective bone. For example, bone graft materials such as bone powders, bone chips, and bone blocks may be used, or methods such as autografting, allografting, and xerografting may be used for reconstruction of defective bone.

Bone graft materials that are used for reconstruction of defective bone may be used in orthopedic surgery, neurosurgery, plastic surgery, otolaryngology, Oral and Maxillofacial Surgery, Department of Veterinary Medicine (veterinary clinic), dermatology and dentistry. For example, these materials may be used for bone defects during disc surgery to induce bone regeneration, or may also be used for implant surgery and reconstruction of oral and maxillofacial bone defects.

Meanwhile, Korean Patent No. 10-0401941 discloses technology related to a bone graft material and a preparation method therefor. When a reticular bone is used which is composed of bioceramic powder and has a three-dimensionally communicating pore structure as disclosed therein, there may be limitations in the effect of bone graft in terms of biocompatibility, mechanical properties, toxicity, and the like.

### SUMMARY

An object of the present disclosure is to provide a bone graft composition containing hydroxypropyl methylcellulose suitable for bone formation, specifically a bone graft composition and a bone graft solution, which form an optimum osmotic pressure with another solution.

One embodiment of the present disclosure provides a bone graft composition with which saline reaches an osmotic pressure of 104 to 112% within 12 to 48 hours after added to a bone graft solution, when the osmotic pressure of the saline is set to 100% as a reference value.

One embodiment of the present disclosure provides a bone graft composition wherein the bone graft solution is a mixture of 1 part by weight of a bone graft material containing hydroxypropyl methylcellulose and 0.5 to 2 parts by weight of a solvent, and the bone graft material containing hydroxypropyl methylcellulose is formed by mixing 1 part by weight of a bone graft material with 0.3 to 3 parts by weight of hydroxypropyl methylcellulose.

One embodiment of the present disclosure provides a bone graft composition wherein the bone graft material is a natural bone graft material.

One embodiment of the present disclosure provides a bone graft composition wherein the solvent is water.

One embodiment of the present disclosure provides a method for preparing a bone graft composition, the method including steps of: (1) preparing a bone morphogenetic protein solution by mixing a solvent and a bone morphogenetic protein; (2) adsorbing the bone morphogenetic protein onto graft material powder by mixing the bone morphogenetic protein and the graft material powder; (3) mixing and stirring the graft material powder having the bone morphogenetic protein adsorbed thereon and hydroxypropyl methylcellulose powder to form a gel that has osmotic properties; and (4) forming a structure containing a plurality of pores by freeze-drying the gel under vacuum.

One embodiment of the present disclosure provides a method for preparing a bone graft composition, wherein the bone morphogenetic protein is at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto.

One embodiment of the present disclosure provides a method for preparing a bone graft composition, wherein the concentration of the bone morphogenetic protein in the bone morphogenetic protein solution is 0.05 to 0.15 mg/ml.

One embodiment of the present disclosure provides a method for preparing a bone graft composition, wherein the pH of the bone morphogenetic protein solution is adjusted to 4.6 to 5 using phosphate buffer saline.

One embodiment of the present disclosure provides a method for preparing a bone graft composition, wherein the volume ratio between the graft material powder having the bone morphogenetic protein adsorbed thereon and the hydroxypropyl methylcellulose powder in step (3) is 1:0.2 to 1:0.6.

One embodiment of the present disclosure provides a method for preparing a bone graft composition, wherein the method further includes a step of sterilizing the bone graft composition by ethylene oxide gas or gamma-ray irradiation.

One embodiment of the present disclosure provides a method for preparing a bone graft composition, wherein the concentration of the ethylene oxide gas is 450 to 1,200 mg/l, or the dose of the gamma-ray irradiation is 10 to 25 kGy.

One embodiment of the present disclosure provides a bone graft composition prepared by the above-described method for preparing a bone graft composition.

### BRIEF DESCRIPTION OF THE DRAWINGS

FIG. 1 is a flow diagram schematically showing a method for preparing a bone graft composition according to one embodiment of the present disclosure.
FIG. 2 shows result data for an Experimental Example of the present disclosure, obtained by mixing saline with bone graft solutions formed using varying amounts (parts by weight) of a solvent, and expressing the time-dependent change in osmotic pressure of the mixed saline compared to that of pure saline as osmotic ratio.

### DESCRIPTION OF SPECIFIC EMBODIMENTS

Embodiments of the present disclosure relate to a bone graft composition which may have excellent effects in terms of activation of bone formation, biocompatibility, and ease of use by containing a porous bone graft material and hydroxypropyl methylcellulose.

However, description of a portion of a particular embodiment, which overlaps with that of other embodiments, will be omitted for a clearer and more concise explanation. Even though description of that portion is omitted, the portion is not excluded from the present disclosure and the scope of rights thereof should be admitted in the same manner as that of other embodiments.

In the following description, the detailed description of publicly-known technology related to the present disclosure will be omitted when it may unnecessarily obscure the subject matter of the present disclosure. In addition, the terms used in the following description are terms defined in consideration of their functions in the present disclosure and may be changed according to the intention of a user or an operator, or according to practice. Therefore, the definitions of these terms should be determined based on the contents throughout the specification.

The technical spirit of the present disclosure is determined by the claims, and the following embodiments are merely means for efficiently explaining the technical spirit of the present disclosure to those skilled in the art to which the present disclosure pertains.

In the present disclosure, when the repeating unit, compound or resin represented by a formula includes isomers thereof, the corresponding formula representing the repeating unit, compound or resin means a representative formula that also represents the isomers.

Hereinafter, specific embodiments of the present disclosure will be described. However, these embodiments are only examples, and the present disclosure is not limited thereto.

The bone graft composition may be implanted into a bone defect, and may be used to restore the bone defect by filling the bone defect. Hereinafter, 'implant' includes being applied into a bone defect in the state of not having rigidity or in the state of having rigdity. Applying into a bone defect in the state of having rigdity may being implanted into a bone defect after being formed the shape corresponding to the shape of the bone defect in the state of having rigdity by a shape forming device, for example 3 dimensional printer.

A bone graft composition of the present disclosure contains a bone graft material and hydroxypropyl methylcellulose. The bone graft composition may be implanted into a bone defect, and may be used to restore the bone defect by filling the bone defect.

The bone graft material may be natural bone, for example, autogenous bone, allogeneic bone, or xenogenic bone. When the natural bone is used, it may exhibit an excellent bone formation effect, because it has excellent biocompatibility and also has good wettability and hygroscopicity due to a large number of pores contained therein. In addition, the natural bone may also be used for reconstruction of defective bone in orthopedic surgery, neurosurgery, plastic surgery, otolaryngology, Oral and Maxillofacial Surgery, Department of Veterinary Medicine (veterinary clinic), dermatology and dentistry.

In addition, the bone graft material may also be used for reconstruction of defective bone in human or animals. Hereinafter, it mainly described the usage to the dentistry, however, the usage does not limited thereto.

As the bone graft composition includes hydroxypropyl methylcellulose, the bone graft composition may have adhesion to a bone defect. When the bone graft composition has excellent adhesion, even if the bone graft composition is applied to the maxilla, it may not flow down, and even if there is an impact due to mastication motion, the bone graft composition may be prevented from being detached from the bone defect.

In addition, as the bone graft composition contains hydroxypropyl methylcellulose, the hydroxypropyl methylcellulose may influence the formation of the osmotic pressure between the composition and another solution. Depending on the degree of dissolution of the hydroxypropyl methylcellulose in a solvent, the osmotic pressure occurring between the composition and another solution varies. Under a condition in which an optimum osmotic pressure is formed, when the bone graft composition is applied to a bone defect, a medical procedure for application of the composition may be more easily performed by minimizing the effect of external conditions.

According to one embodiment of the present disclosure, a bone graft composition showing optimal bone graft properties during a medical procedure may contain hydroxypropyl methylcellulose in an amount of 0.15 to 6 parts by weight, more preferably 0.3 to 3 parts by weight, based on 1 part by weight of the bone graft material. If the content of the hydroxypropyl methylcellulose is less than 0.3 parts by weight based on 1 part by weight of the bone graft material, the composition has insufficient adhesion to a bone defect, and hence is highly likely to detach from the bone defect during use, and the hydroxypropyl methylcellulose has little or no effect on the osmotic properties of the bone graft composition because the content thereof is insignificant. If the content of the hydroxypropyl methylcellulose is more than 3 parts by weight per part by weight of the bone graft material, the hydroxypropyl methylcellulose may interfere with bone formation by interfering with the wettability, hygroscopicity or the like of xenogenic bone, and hydroxypropyl methylcellulose may not easily dissolve due to curing thereof, and may have a great effect on the osmotic pressure of the composition due to flowing out thereof, and thus the composition may not suitably function as a bone graft material.

The bone graft composition containing the hydroxypropyl methylcellulose is used as a solution obtained by dissolving it in a solvent. As the solvent, any solvent that may be used in the art may be suitably selected and used, and for example, water is used. Since the physical properties of the bone graft composition, such as dissolution rate, concentration, osmotic properties, and shape retainability, vary depending on the conditions of the solvent, it is important to select suitable solvent conditions for the practice of the present disclosure.

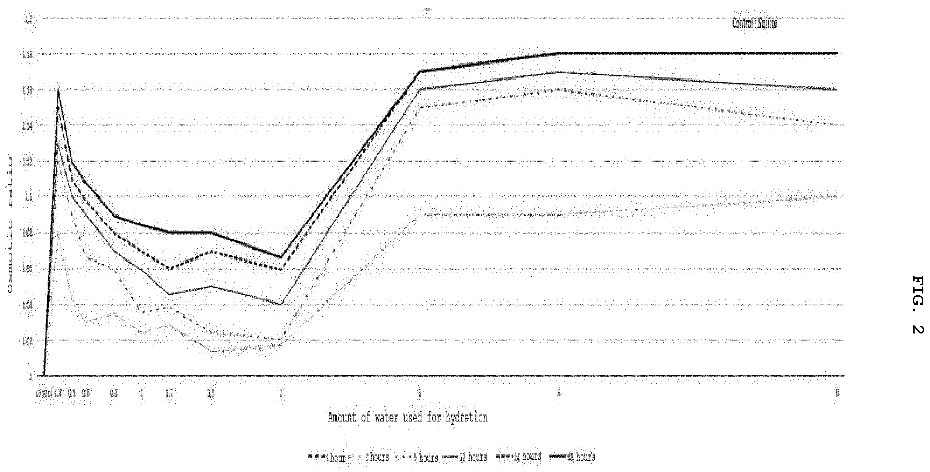

According to one embodiment of the present disclosure, the bone graft solution may be prepared by mixing 1 part by weight of the bone graft composition containing hydroxypropyl methylcellulose with 0.5 to 2 parts by weight of the solvent (water), in order for the bone graft solution to show optimal properties during a medical procedure, specifically in order for the bone graft solution to form an optimum osmotic pressure with another solution. As can be seen from the Experimental Example to be described later, the content of the solvent should be within a certain range so that the bone graft composition solution can form an appropriate osmotic phenomenon with another solution. As shown in FIG. 2, it can be seen that 0.5 to 2 parts by weight of the solvent (water) should be mixed with 1 part by weight of the bone graft composition containing hydroxypropyl methylcellulose, in order to form an appropriate osmotic phenomenon.

If the content of the solvent (water) is less than 0.5 parts by weight based on 1 part by weight of the bone graft composition containing hydroxypropyl methylcellulose, dissolution may not easily occur because the amount of the solvent (water) is excessively small, and hence the hydroxypropyl methylcellulose may not easily bond with the bone graft material and may not sufficiently exhibit its function. Generally, the composition may be so stiff that it cannot be suitable for bone formation. In addition, it may form a strong osmotic pressure with another solution, and thus the bone graft material may not retain its shape and the function thereof can be impaired.

If the content of the solvent (water) is more than 2 parts by weight based on 1 part by weight of the bone graft composition containing hydroxypropyl methylcellulose, the amount of the solvent (water) is so large that the solution has a property of flowing without having proper viscosity, and thus the composition may not have shape retainability. In addition, dissolution of the hydroxypropyl methylcellulose may proceed rapidly due to a high content of the solvent (water), and thus the hydroxypropyl methylcellulose may flow out so that it cannot exhibit its function. In addition, it may form a strong osmotic pressure with another solution, and thus the bone graft material may not retain its shape and the function thereof can be impaired.

Thus, as a solvent condition for allowing the bone graft composition containing hydroxypropyl methylcellulose to sufficiently exhibit the function of the hydroxypropyl methylcellulose and to retain the shape of the bone graft material, 0.5 to 2 parts by weight of the solvent (water) is preferably mixed with 1 part by weight of the bone graft composition containing hydroxypropyl methylcellulose to prepare a bone graft solution.

A bone graft composition kit according to another embodiment of the present disclosure includes the above-described bone graft composition and a syringe containing the composition. By providing the syringe directly containing the bone graft composition, it is possible to ensure ease of use and significantly reduce the possibility of contamination that may occur during use.

However, in the description of this embodiment, the description of a portion that overlaps with that of other embodiments is omitted for a clearer and more concise explanation. Even though the description of that portion is omitted, the portion is not excluded from the present disclosure and the scope of rights thereof should be admitted in the same manner as that of other embodiments.

A method for preparing a bone graft composition according to still another embodiment of the present disclosure includes steps of: preparing a bone morphogenetic protein solution by adding a bone morphogenetic protein to a solvent or adding the bone morphogenetic protein to the solvent to dissolve the bone morphogenetic protein in the solvent;

mixing and stirring graft material powder having the bone morphogenetic protein adsorbed thereon and hydroxypropyl methylcellulose powder to form a viscous gel; and

forming a sponge-like structure containing a plurality of pores by freeze-drying the mixture of the graft material powder and the hydroxypropyl methylcellulose powder, obtained by the mixing and stirring process, at low temperature under vacuum. The bone graft composition prepared through these steps may have excellent effects in terms of activation of bone formation, biocompatibility, and ease of use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-described embodiments.

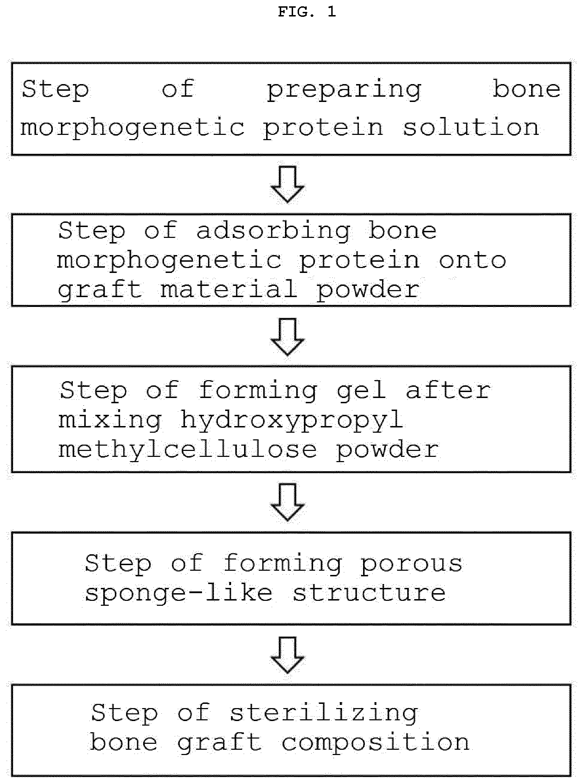

FIG. 1 is a flow diagram schematically showing a method for preparing a bone graft composition according to one embodiment of the present disclosure.

FIG. 2 shows result data for an Experimental Example of the present disclosure, obtained by mixing saline with bone graft solutions formed using varying amounts (parts by weight) of a solvent, and then expressing the time-dependent change in osmotic pressure of the mixed saline compared to that of pure saline as osmotic ratio.

First, a bone morphogenetic protein solution is prepared by dissolving a bone morphogenetic protein in a solvent. The bone morphogenetic protein solution may be prepared by adding the bone morphogenetic protein to the solvent, or adding the bone morphogenetic protein to the solvent and dissolving the bone morphogenetic protein in the solvent.

The bone morphogenetic protein may be at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto. Preferably, the bone morphogenetic protein may be rhBMP-2 in terms of the bone formation effect of the present disclosure.

According to one embodiment of the present disclosure, the concentration of the bone morphogenetic protein in the bone morphogenetic protein solution may be 0.05 to 0.15 mg/ml, preferably 0.08 to 0.12 mg/ml. When the concentration of the bone morphogenetic protein is within the above-described range, bone formation by the bone morphogenetic protein may be activated. If the concentration of the bone morphogenetic protein is less than 0.05 mg/ml, the ability of the bone morphogenetic protein to form new bone may be reduced, and if the concentration of the bone morphogenetic protein is more than 0.15 mg/ml, it may cause adverse effects.

In addition, according to one embodiment of the present disclosure, the pH of the bone morphogenetic protein solution may be, for example, 4.6 to 5. When the pH is within the above-described range, bone formation by the bone morphogenetic protein may be activated. If the pH of the bone morphogenetic protein solution is less than 4.6, the ability to form new bone may be reduced, and if the pH of the bone morphogenetic protein solution is more than 5, the ability to form new bone may be reduced. For example, the pH may be adjusted using phosphate buffer saline. When the pH is adjusted using phosphate buffer saline, the bone morphogenetic protein may have the effect of forming new bone.

Thereafter, the bone morphogenetic protein is adsorbed onto graft material powder by soaking the graft material powder with the bone morphogenetic protein solution. The previously prepared graft material powder may be soaked with the bone morphogenetic protein solution by flushing the graft material powder with the bone morphogenetic protein solution or dropping the graft material powder into the bone morphogenetic protein solution, whereby the bone morphogenetic protein may be adsorbed onto the graft material powder.

The graft material powder may be autogenous bone, allogeneic bone, or xenogenic bone. For example, the graft material powder may be prepared by placing it in a snap tube.

The average particle diameter (D50) of the graft material powder may be 200 to 5,000 µm, preferably 250 to 1,000 µm. If the average particle diameter of the powder is less than 200 µm, the graft material may be absorbed rapidly, and thus osteoconduction required for bone formation may be insufficient, and if the average particle diameter of the powder is more than 5,000 µm, precise processing of the graft material powder during application to a patient may be difficult.

According to one embodiment of the present disclosure, the step of adsorbing the bone morphogenetic protein onto the graft material powder may include a step of adsorbing the bone morphogenetic protein using a refrigerated centrifuge.

In some cases, the bone morphogenetic protein may also be suspended in the solution. However, when the bone morphogenetic protein is adsorbed while it is rotated at high speed using a centrifuge, the bone morphogenetic protein can be prevented from being suspended in the solution, and thus the bone morphogenetic protein may be easily adsorbed onto the surface or into the pores of the graft material powder. Only when the bone morphogenetic protein is adsorbed while it is rotated at high speed, it can be prevented from being suspended again after detachment from the graft material powder. If the bone morphogenetic protein is rotated at low speed, it can be suspended, and hence cannot be easily adsorbed. Under highspeed rotation, the bone morphogenetic protein can be adsorbed quickly onto the surface or into the pores of the graft material powder.

According to one embodiment of the present disclosure, the rotational speed of the refrigerated centrifuge may be 4,000 rpm or more. When the bone morphogenetic protein is adsorbed using the centrifuge, the higher the rotational speed, the better the adsorption. For example, the rotational speed of the centrifuge may be 4,000 rpm or more, and when this rotational speed range is satisfied, the bone morphogenetic protein can be prevented from being suspended in the solution.

According to one embodiment of the present disclosure, the step of adsorbing the bone morphogenetic protein using the refrigerated centrifuge may be performed at a cold temperature of 5°C or below. As the step of adsorbing the bone morphogenetic protein using the refrigerated centrifuge is performed at a cold temperature of 5°C or below, it is possible to maximize the effect of adsorbing the bone morphogenetic protein onto the surface or into the pores of the graft material powder through rotation while preventing the denaturation of the bone morphogenetic protein that is weak to heat, by preventing the temperature of the solution from being increased due to rotation. The cold temperature may be a temperature at which the solution does not freeze. For example, the cold temperature may be 5°C or below, preferably 0.5 to 1.5°C.

Thereafter, the graft material powder having the bone morphogenetic protein adsorbed thereon and hydroxypropyl methylcellulose powder are mixed and stirred to form a gel. The viscous gel thus formed can improve the adhesion of the graft material powder. For example, the stirring may be performed using a mixer. As the graft material powder is stirred with the hydroxypropyl methylcellulose in powder form, a product with homogeneous quality can be obtained.

According to one embodiment of the present disclosure, the volume ratio between the graft material powder having the bone morphogenetic protein adsorbed thereon and the hydroxypropyl methylcellulose powder may be 1:0.2 to 1:0.6. If the volume ratio of the hydroxypropyl methylcellulose powder is less than 0.2, it may be difficult to form a gel, and if the volume ratio of the hydroxypropyl methylcellulose powder is more than 0.6, it may be difficult to form an effective bone graft composition because the volume of the gel is larger than the volume of the graft material powder. In terms of the effects of the present disclosure, the volume ratio between the graft material powder having the bone morphogenetic protein adsorbed thereon and the hydroxypropyl methylcellulose powder may preferably be 1: 0.25 to 1: 0.35.

Thereafter, the mixture of the graft material powder and the hydroxypropyl methylcellulose powder, obtained by the mixing and stirring process, is freeze-dried under vacuum to form a sponge-like structure containing pores. A sponge-like structure containing a plurality of pores may also be formed by freeze-drying the mixture of the graft material powder and the hydroxypropyl methylcellulose powder, obtained by the mixing and stirring process, at a low temperature under vacuum.

A sponge-like structure including a porous structure may be formed by the freeze-drying treatment under vacuum. The gel may be absorbed into the graft material powder to form a sponge-like structure including a porous structure, and it is believed that the treatment under vacuum mainly contributes to the formation of the sponge-like structure including a porous structure.

According to one embodiment of the present disclosure, the method for preparing the bone graft composition may further include a packaging step.

According to one embodiment of the present disclosure, the method for preparing the bone graft composition may further include a step of placing the prepared bone graft composition including a sponge-like structure containing a plurality of pores in a snap tube sized to be inserted into a syringe. When the method further includes the step of placing the composition in a snap tube sized to be inserted into a syringe, the composition may be sized to be inserted into the syringe and thus may be inserted directly into the syringe without a separate process, so that the operation of the process for preparing the bone graft composition can be facilitated.

According to an embodiment of the present disclosure, the method for preparing the bone graft composition may further include a step of placing and sealing the bone graft composition including a sponge-like structure containing a plurality of pores, placed in the snap tube, in a syringe. When the bone graft composition is provided in the syringe, it is possible to ensure ease of use and significantly reduce the possibility of contamination that may occur during use.

According to one embodiment of the present disclosure, the method for preparing the bone graft composition may further include a step of sterilizing the composition.

In one embodiment of the present disclosure, the bone graft composition including a sponge-like structure containing a plurality of pores may be sterilized by ethylene oxide gas. For example, the concentration of the ethylene oxide gas may be 450 to 1,200 mg/l.

If the concentration of the ethylene oxide gas is less than 450 mg/l, sterilization may be insufficient, and if the concentration of the ethylene oxide gas is more than 1,200 mg/l, denaturation of the bone morphogenetic protein may occur.

According to one embodiment of the present disclosure, the bone graft composition including a sponge-like structure containing a plurality of pores may be sterilized by gamma-ray irradiation. For example, the dose of the gamma-ray irradiation may be 10 to 25 kGy. If the dose of the gamma-ray irradiation is less than 10 kGy, sterilization may be insufficient, and if the dose of the gamma-ray irradiation is more than 25 kGy, denaturation of the bone morphogenetic protein may occur.

The bone graft composition prepared according to the above-described method should have an ensured hydration ability for application of the composition to the human body, for example, application of the composition to teeth. In other words, the bone graft composition should have critical osmotic properties. Allowing the bone graft composition to have a certain osmotic ratio when mixed with another solution (e.g., saline) is an important factor in the ability of the bone graft material to be maintained at a constant concentration to thereby retain its shape and function. This property may be determined according to the content of HPMC or the like contained in the bone graft composition, as well as the condition of the solvent for forming the solution.

For example, in the case in which the bone graft composition is applied to teeth, a dental operator applies the bone graft composition, which is provided as powder or the like, to a missing tooth after hydrating the composition. If the HPMC contained in the bone graft composition flows out in large amounts (that is, the osmotic pressure of the hydrated composition increases) during this hydration process, the HPMC cannot remain inside the bone graft material, and thus the bone graft material cannot retain its shape and also cannot agglomerate, making it impossible to perform the medical procedure. For this reason, the bone graft composition should have an osmotic properties within a predetermined range so that it can retain its shape without being influenced by external conditions. Furthermore, if an external environment such as water or saliva is formed after the bone graft composition is applied to the missing tooth, a phenomenon may occur in which the bone graft composition flows out around or detaches, and thus a problem may arise in that an external substance flows into the missing tooth. For this reason, the bone graft composition should have an osmotic properties within a predetermined range, and furthermore, have not only internal physical properties, but also properties that resist external conditions.

Actually, when the osmotic pressure of saline is set to 100% as a reference value, the saline should reach an osmotic pressure of 104 to 112% within 12 to 48 hours after the saline is added to the bone graft solution. If the osmotic pressure is more than 112%, the HPMC may flow out during the hydration process, and thus the shape retainability of the bone graft composition and the adhesion thereof to a bone defect may significantly decrease, making it impossible to apply the bone graft composition. On the other hand, if the osmotic pressure is less than 104%, the bone graft composition may not be easily hydrated, and thus may be difficult to deform plastically to the bone defect, thus adversely affecting bone regeneration.

Hereinafter, preferred examples will be presented to help the understanding of the present disclosure. However, these examples are merely to illustrate the present disclosure and are not intended to limit the scope of the present disclosure as defined in the appended claims. In addition, it will be obvious to those skilled in the art that various changes and modifications of these examples are possible without departing from the scope and technical spirit of the present disclosure. In addition, it is to be understood that these changes and modifications also fall within the appended claims.

### Experimental Examples

### 1. Measurement of Osmotic Pressure of Saline

An osmotic pressure refers to a pressure that is applied to semipermeable membrane when an osmotic phenomenon occurs, and it is proportional to the difference in the solution concentration. In a control experiment, the osmotic pressure of saline was measured, and as shown in Table 1 below, whether the osmotic pressure changed over time was also measured. The osmotic pressure can be obtained by introducing a solution and measuring the osmotic pressure value by a pressure sensor connected to an outlet. The osmotic pressure value of saline measured in this control experiment was 286, which was set to 100% as a reference value.

2. Preparation of Hydrated Bone Graft Materials Containing Hydroxypropyl Methylcellulose (HPMC)

Hydrated bone graft materials containing hydroxypropyl methylcellulose (HPMC) were prepared by adding water to 1 part by weight (0.5 g) of a bone graft material and 0.6 parts by weight of HPMC. The amount of water added was 0.4 to 6 parts by weight per part by weight of the mixture of the bone graft material and HPMC.

3. Measurement of Osmotic Pressure of Saline Added to Hydrated Bone Graft Materials Containing Hydroxypropyl Methylcellulose (HPMC)

Each of the hydrated bone graft materials prepared in the above section 2 was placed in a 15-ml conical tube, and 5 ml of saline was added thereto. At each of the time points shown in Table 1 below, 0.5 ml of the saline was taken and the osmotic pressure was measured. The measured osmotic pressure value was expressed as a percentage (%) relative to the reference value obtained in the control experiment of the above section 1.

**Table 1**

| | 1 hour | 3 hours | 6 hours | 12 hours | 24 hours | 48 hours |
|---|---|---|---|---|---|---|
| Control | 100% | 100% | 100% | 100% | 100% | 100% |
| 0.4 | 100% | 108% | 112% | 113% | 115% | 117% |
| 0.5 | 100% | 104% | 109% | 110% | 111% | 112% |
| 0.6 | 100% | 103% | 107% | 109% | 110% | 111% |
| 0.8 | 100% | 103% | 106% | 107% | 108% | 109% |
| 1.0 | 100% | 102% | 103% | 106% | 107% | 108% |
| 1.2 | 100% | 103% | 104% | 105% | 106% | 108% |
| 1.5 | 100% | 101% | 102% | 105% | 107% | 108% |
| 2.0 | 100% | 102% | 102% | 104% | 106% | 107% |
| 3.0 | 100% | 109% | 115% | 116% | 117% | 117% |
| 4.0 | 100% | 109% | 116% | 117% | 118% | 118% |
| 6.0 | 100% | 110% | 114% | 116% | 118% | 118% |

As shown in Table 1 above, the value obtained in the control experiment was set to 100% as a reference value. This value is a value obtained by standardizing the osmotic pressure of pure saline, measured at each time point.

As shown in Table 1 above, it can be confirmed that when the amount (parts by weight) of water added was constant, the osmotic pressure increased over time. This suggests that the time and amount of contact between the saline and the hydrated bone graft material containing dissolved HPMC increased over time, thus increasing the osmotic pressure.

As shown in Table 1 above, it can be confirmed that, at the same time point, the osmotic pressure increased as the amount (parts by weight) of water added increased. This suggests that, as the amount of water added increased, the HPMC in the hydrated bone graft material containing dissolved HPMC dissolved more quickly, thus increasing the osmotic pressure.

As shown in FIG. 2, it can be confirmed that when the amount of water added was 0.4 parts by weight or less, the osmotic pressure greatly increased within a short time, and thus the osmotic ratio greatly changed, and even when the amount of water added was 3 parts by weight or more, the osmotic pressure greatly increased within a short time, and thus the osmotic ratio greatly changed. The great increase in the osmotic pressure within a short time means that the HPMC was not fused with the bone graft material and did flow out, and thus the osmotic pressure increased with increasing concentration, leading to a decrease in the shape retainability. Therefore, in order to optimize the shape retainability, the amount of water added is preferably set to 0.5 to 2 parts by weight based on 1 part by weight of the bone graft composition containing HPMC.

When the amount of water added is 0.5 to 2 parts by weight based on 1 part by weight of the bone graft composition, the osmotic pressure of the saline may reach 104 to 112% within 12 to 48 hours. It is insufficient to understand performance on the functional maintenance of the bone graft composition from the osmotic pressure value that is reached within 12 hours, and when this value is applied as it is, the demonstration of the functionality and stability of the composition as a bone graft material will be somewhat insufficient. Therefore, by examining the osmotic pressure that is reached within 12 to 48 hours, it is possible to determine the functionality, osmotic properties, shape retainability and the like of the bone graft material. If the osmotic pressure within 48 hours exceeds 115% of the initial osmotic pressure of saline, it means that hydration of the bone graft composition containing HPMC did not occur well. In this case, the water does not sufficiently function as an additive, and thus the composition is difficult to use as a bone graft composition. If the osmotic pressure within 48 hours is close to 100%, it is not different from the osmotic pressure of saline, and thus it is hardly considered that the bone graft composition containing hydroxypropyl methylcellulose (HPMC) has the desired properties and functions. Therefore, it is preferable to prepare a bone graft composition with which saline reaches an osmotic pressure of 104 to 112% of the osmotic pressure, obtained in the control experiment, within 12 hours to 48 hours after addition thereof.

As described above, the bone graft composition forms an optimum osmotic pressure with another solution, and thus has excellent effects in terms of activation of bone, biocompatibility, and ease of use.

## Claims

1. A bone graft composition with which saline reaches an osmotic pressure of 104 to 112% within 12 to 48 hours after added to a bone graft solution, when an osmotic pressure of the saline is set to 100% as a reference value.

2. The bone graft composition of claim 1, wherein the bone graft solution is a mixture of 1 part by weight of a bone graft material containing hydroxypropyl methylcellulose and 0.5 to 2 parts by weight of a solvent, and the bone graft material containing hydroxypropyl methylcellulose is formed by mixing 1 part by weight of a bone graft material with 0.3 to 3 parts by weight of hydroxypropyl methylcellulose.

3. The bone graft composition of claim 1, wherein the bone graft material is a natural bone graft material.

4. The bone graft composition of claim 2, wherein the solvent is water.

5. A method for preparing a bone graft composition, the method comprising steps of:
(1) preparing a bone morphogenetic protein solution by mixing a solvent and a bone morphogenetic protein;
(2) adsorbing the bone morphogenetic protein onto graft material powder by mixing the bone morphogenetic protein and the graft material powder;
(3) mixing and stirring the graft material powder having the bone morphogenetic protein adsorbed thereon and hydroxypropyl methylcellulose powder to form a gel that has osmotic properties; and
(4) forming a structure containing a plurality of pores by freeze-drying the gel under vacuum.

6. The method of claim 5, wherein the bone morphogenetic protein is at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto.

7. The method of claim 5, wherein a concentration of the bone morphogenetic protein in the bone morphogenetic protein solution is 0.05 to 0.15 mg/ml.

8. The method of claim 5, wherein a pH of the bone morphogenetic protein solution is adjusted to 4.6 to 5 using phosphate buffer saline.

9. The method of claim 5, wherein a volume ratio between the graft material powder having the bone morphogenetic protein adsorbed thereon and the hydroxypropyl methylcellulose powder in step (3) is 1:0.2 to 1:0.6.

10. The method of claim 5, further comprising a step of sterilizing the bone graft composition by ethylene oxide gas or gamma-ray irradiation.

11. The method of claim 5, wherein a concentration of the ethylene oxide gas is 450 to 1,200 mg/l, or a dose of the gamma-ray irradiation is 10 to 25 kGy.
